# EUROPÄISCHE PATENTSCHRIFT

(11) **EP 1 633 115 B1**
(45) Veröffentlichungstag und Bekanntmachung des Hinweises auf die Patenterteilung: **05.11.2008**
(21) Anmeldenummer: 05018977.8
(22) Anmeldetag: 01.09.2005
(51) Int. Cl.: H04L 29/08, A61B 5/00, A61N 1/372

(54) **Vorrichtung und Verfahren zum Betreiben eines mobilen Kommunikationsgerätes**
Apparatus and method for operating a mobile communication device
Appareil et procédé pour faire fonctionner un dispositif de communication mobile

(30) Priorität: 03.09.2004 DE 102004043211
(43) Veröffentlichungstag der Anmeldung: 08.03.2006
(73) Patentinhaber: BIOTRONIK CRM Patent AG, 6340 Baar/Zug (CH)
(72) Erfinder: Behrens, Günter, 12101 Berlin (DE); Bode, Sven, Dr., 12203 Berlin (DE); Diebold, Michael, 12169 Berlin (DE); Potschadtke, Jens, 91056 Erlangen (DE)
(74) Vertreter: Lindner-Vogt, Karin L.

(56) Entgegenhaltungen:
- WO-A-00/41486
- WO-A-03/095024
- WO-A-20/04066656
- US-B1- 6 516 188
- "Revision of the Commission's rules to ensure compatibility with enhanced 911 emergency calling systems" REPORT AND ORDER BEFORE THE FEDERAL COMMUNICATIONS COMMISION WASHINGTON, D.C., 13. Mai 1999 (1999-05-13), XP002280299

## Beschreibung

Die Erfindung betrifft eine Vorrichtung mit wenigstens einer drahtlosen Datenschnittstelle (Telemetriedatenschnittstelle), die als Mobilfunkschnittstelle ausgelegt ist, einen Datenaustausch über ein Mobilfunk- oder drahtloses Datennetz durchzuführen, sowie mit einem Benutzeridentifikationsmodul, die Informationen zum Betrieb enthält, die unter anderem die Berechtigung eines Benutzers zur Benutzung von Mobilfunk- oder drahtlosen Datennetzen ausweisen, und mit einer Kontrolleinheit, die auf Basis der auf dem Benutzeridentifikationsmodul gespeicherten Informationen Zustandekommen und Durchführung des Datenaustausches steuert.

Weiterhin betrifft die vorliegende Patentanmeldung ein Verfahren zum Betreiben einer solchen Vorrichtung.

Derartige Vorrichtungen sind prinzipiell im Stand der Technik bekannt und werden z. B. in der medizinischen Überwachung (Home Monitoring) von Risikopatienten oder der Fernwartung und -diagnose von technischen Einrichtungen eingesetzt, indem sie einer Datenerfassungs- und -auswertezentrale technische oder medizinische Daten über ein Mobilfunk- oder drahtloses Datennetz übermitteln und ggf. von dort Anweisungen entgegennehmen.

Für das Home Monitoring von Risikopatienten werden dabei oft tragbare Ausführungen bevorzugt, die über eine zweite Telemetrieschnittstelle zur Kommunikation mit einem elektromedizinischen Implantat verfügen. Moderne elektromedizinische Implantate, insbesondere Herzschrittmacher, Defibrillatoren und dergleichen, bieten Arzt und Patienten ein Höchstmaß an Sicherheit und Komfort durch diese Home Monitoring Funktionen.

Dabei protokolliert das Implantat Diagnose- und Therapieinformationen und überträgt diese Informationen über eine Telemetrieschnittstelle an ein externes Patientengerät. Von dort werden die Daten an ein sog. Home Monitoring Service Center (HMSC) weitergeleitet, wo sie für den Arzt gespeichert und visualisiert werden. Der Arzt kann sich auf diese Weise direkt über den Therapieverlauf und den aktuellen Gesundheitszustand seiner Patienten informieren und bekommt die Möglichkeit, schnell auf eventuelle Gesundheitsveränderungen zu reagieren.

Ohne Home Monitoring kann der Arzt diese Informationen nur im Rahmen einer Untersuchung des Patienten abfragen. In kritischen Situationen würde dies zu unerwünschten Verzögerungen im Informationsfluss führen. Zudem ist jede Untersuchung für Arzt und Patient mit erheblichem Zeitaufwand verbunden. Häufige Untersuchung beeinträchtigen Mobilität und Lebensqualität des Patienten.

Beim Home Monitoring werden die Implantatinformationen über das Patientengerät (siehe auch US 6,553,262, US 5,752,976, WO 03/095024) im Hintergrund verschickt, ohne dass der Patient in der normalen Lebensführung eingeschränkt wird; d.h., er genießt die Sicherheit des ärztlichen Monitorings ohne die Belastung von häufigen Untersuchungen.

Im Falle einer technischen Einrichtung können durch die fortlaufende Überwachung besondere Betriebszustände erkannt werden, so dass deren Weiterbetrieb gewährleistet werden kann.

Da die Bewegungsfreiheit eines Patienten nicht unnötig eingeschränkt werden soll, wird die Datenübertragung bevorzugt über eines der flächendeckend verfügbaren Mobilfunknetze, insbesondere das GSM-, das UMTS- oder ein CDMA-Netz vorgenommen; auch eine Datenübertragung über ein WLAN-Netz ist vorstellbar. Bei einer technischen Einrichtung steht eher der Vorteil der einfachen Anbindung über eines der genannten drahtlosen Kommunikationsnetze im Vordergrund, jedoch sind auch bewegliche Einrichtungen vorstellbar, für die ebenfalls der Gesichtspunkt der uneingeschränkten Mobilität anzuführen ist. So könnte ein Gerät der beanspruchten Art in Fahrzeugen im Lieferbetrieb oder Personentransport eingesetzt werden, um z. B. Aufenthaltsort, Beladung und technische Daten wie den Tankfüllstand oder die Temperatur des Laderaumes an eine Leitzentrale zu übermitteln. In diesem Fall sind Fahrzeug und beanspruchtes Gerät gewöhnlich zu einer Einheit verbunden.

Die Entwicklung eines Gerätes der beschriebenen Art mitsamt der Mobilfunkschnittstelle ist sehr aufwendig. Deshalb wird es in der Praxis zum Teil aus vorgefertigten Modulen aufgebaut, die bestimmte Funktionen zur Verfügung stellen. Dies trifft insbesondere auf die Mobilfunkschnittstelle zu, die normalerweise durch ein in das Gerät eingebautes Mobiltelefon oder ein vorgefertigtes Mobilfunkmodul mit vollem Funktionsumfang verwirklicht wird.

Um die Zugangsberechtigung zu einem drahtlosen Kommunikationsnetz nachzuweisen, werden mit einer Mobilfunkschnittstelle ausgestattete Geräte mit einem Benutzeridentifikationsmodul ausgestattet, die das Gerät bzw. dessen Betreiber aufgrund von auf dem Benutzeridentifikationsmodul gespeicherter Informationen eindeutig identifiziert. Diese Informationen übermittelt das Gerät bei der Anmeldung zu einem drahtlosen Kommunikationsnetz an dieses Netz, welches dann durch Vergleich mit einer zentral gespeicherten Kopie die Zugangsberechtigung des Gerätes überprüft. Die Identifikation ist u. a. deshalb notwendige Voraussetzung der Benutzung eines mobilen Dienstes, da nur durch sie die anfallenden Verbindungskosten abgerechnet werden können. Deshalb wird sie auch beim sog. "Roaming", also dem Nutzen von Netzressourcen und -diensten von Mobilfunknetzbetreibern anderer Länder, immer vorgenommen. Bei einem Gerät, das ausgelegt ist, über ein GSM-Netz zu kommunizieren, ist das Benutzeridentifikationsmodul als sog. SIM-Karte ausgeführt, die Kopie der Zugangsberechtigung ist im sog. "Home-Location Register" (HLR) abgelegt.

Kann eine Zugangsberechtigung zum fraglichen drahtlosen Kommunikationsnetz nicht festgestellt werden, übermittelt das drahtlose Kommunikationsnetz dem Gerät, das die Anmeldung versucht, die Aufforderung, weitere Anmeldeversuche bei demselben Netz zu unterlassen, um dessen Ressourcen zu schonen. Mobilfunktelefone und Mobilfunkmodule beachten standardmäßig diese Information über eine Zurückweisung und unterlassen weitere Anmeldeversuche bei dem fraglichen Kommunikationsnetz. Ein neuerlicher Anmeldeversuch kann nur durch Bedienung der Tastatur des Mobiltelefons bzw. des Mobilfunkmoduls ausgelöst werden. Ist keine Tastatur vorgesehen oder diese unzugänglich, kann eine neuerliche Anmeldeprozedur gar nicht ausgelöst werden.

Ein Anmeldeversuch bei einem Kommunikationsnetz wird von diesem auch dann zurückgewiesen, wenn eine Überprüfung der Zugangsberechtigung nicht möglich ist. Das passiert z. B. dann, wenn eine Verbindung zum HLR aufgrund einer Störung nicht hergestellt werden konnte. Solche Zugriffe können insbesondere beim Roaming wegen des komplizierteren Zugriffs auf das HLR gestört werden.

Problematisch ist nun, dass es unter Umständen dazu kommen kann, dass ein Gerät von allen empfangbaren Netzen zurückgewiesen wird und von sich aus alle weiteren Versuche, eine Verbindung über ein drahtloses Kommunikationsnetz herzustellen, einstellt. Es kann dann nur durch äußeren Eingriff zu neuerlichen Anmeldeversuchen bewegt werden. Da aber entweder der Benutzer des Gerätes nicht durch eine komplizierte Benutzerschnittstelle behelligt werden soll oder aber überhaupt keine Person vor Ort anwesend ist, kann der Betrieb eines Gerätes der beschriebenen Art nach dem Stand der Technik nicht mehr gewährleistet werden.

Die Dokumente WO 00/41486, XP 002280299, WO 2004/066656 und US 6516188 offenbaren auch das oben genannte Problem.

Aufgabe der vorliegenden Erfindung ist deshalb, ein Gerät und ein Verfahren zum Betrieb eines solchen Gerätes einzuführen, das auch nach Zurückweisung durch alle empfangbaren drahtlosen Kommunikationsnetze eine Kommunikation des Gerätes mit dem Home Monitoring Service Center ermöglicht, wenn die Ursache der Zurückweisung nicht mehr besteht.

Erfindungsgemäß wird diese Aufgabe durch ein Gerät, in Anspruch 1, der eingangs genannten Art gelöst, das eine zweite Kontrolleinheit aufweist, die ausgebildet ist, eine durch die örtlich verfügbaren Mobilfunk- oder drahtlosen Datennetze ausgesprochene Zurückweisung des Benutzeridentifikationsmoduls bei der Anmeldung zum Mobilfunk- oder drahtlosen Datennetz durch geeignete Steuerung der ersten Kontrolleinheit im weiteren Verlauf des Anmeldeverfahrens durch Umgehung oder Löschen übermittelter Information über die Zurückweisung unwirksam macht oder in der Dauer ihrer Wirkung beschränkt.

In einer besonders bevorzugten Ausführung des Gerätes ist dieses tragbar.

In einer bevorzugten Ausführung verfügt das Gerät über eine zweite Telemetriedatenschnittstelle, über die es mit einem elektromedizinischen Implantat, insbesondere einem Herzschrittmacher, kommunizieren kann, und durch Auswerten und/oder Weiterleiten der von dem elektromedizinischen Implantat empfangenen technischen und medizinischen Daten über die erste Telemetriedatenschnittstelle zu einem HMSC ein Home Monitoring eines Patienten ermöglicht.

In weiteren bevorzugten Ausführungen wird die Kommunikationsverbindung über die Mobilfunkschnittstelle alternativ über ein GSM-, ein UMTS-, ein CDMA- oder ein WLAN-Netz vorgenommen. Dabei kann das Gerät über eine beliebige Auswahl an Schnittstellen zu Netzen der genannten Arten verfügen. Dies erleichtert die Anpassung des Gerätes an die Gegebenheiten des jeweiligen Marktes und erhöht die Wahrscheinlichkeit einer Netzanbindung in der entsprechenden Umgebung.

Mindestens eine der Kontrolleinheiten des Gerätes ist vorzugsweise dazu ausgebildet, eine erfolglose Anmeldung wegen Zurückweisung durch sämtliche vor Ort verfügbaren Mobilfunk- oder drahtlosen Datennetze zu erkennen. Daraufhin können die besonderen Schritte des beanspruchten Verfahrens eingeleitet werden.

In einer weiteren bevorzugten Ausführung ist die erste Kontrolleinheit ausgebildet, eine Liste der lokal empfangbaren Mobilfunk- oder drahtlosen Datennetze zu erstellen und der zweiten Kontrolleinheit zugänglich zu machen. In einer besonders bevorzugten Variante dieser Ausführung ist die zweite Kontrolleinheit dazu ausgebildet, nach einer gescheiterten Anmeldung ein anderes Netz von der Liste auszuwählen und die erste Kontrolleinheit zu einem Anmeldeversuch bei diesem Netz zu veranlassen, selbst wenn dieses Netz eine Anmeldung bei einem vorhergehenden Versuch bereits zurückgewiesen hatte. In einer besonders bevorzugten Variante dieser Ausführung ist die zweite Kontrolleinheit ausgebildet, nach erfolglosen Anmeldeversuchen bei allen Netzen der Liste eine neue Liste von der ersten Kontrolleinheit erstellen zu lassen.

In einer weiteren bevorzugten Variante enthält die zweite Kontrolleinheit einen Zeitgeber oder ist mit einem solchen verbunden. Die Kontrolleinheit ist derart ausgebildet, dass sie nach einer gescheiterten Anmeldung eine vorgegebene, durch den Zeitgeber gemessene Zeitdauer verstreichen lässt, bevor sie einen neuerlichen Anmeldeversuch bei demselben Netz einleitet. Dadurch werden sowohl die Ressourcen der Netzbetreiber als auch das Batterieleben der Vorrichtung geschont.

In einer bevorzugten Ausführungsform sind die Kontrolleinheiten ausgeführt, AT-Befehle auszugeben und/oder auszuführen. AT-Befehle stellen einen allgemein akzeptierten Standard dar, weshalb viele vorgefertigte Komponenten und Module mit ihnen arbeiten; ihre Benutzung vereinfacht deshalb den Aufbau des Gesamtsystems.

In einer weiteren bevorzugten Ausführung enthält die zweite Kontrolleinheit einen Zeitgeber oder ist mit einem solchen verbunden. Sie ist derart ausgebildet, dass sie wiederholte, bei allen verfügbaren Mobilfunk- oder drahtlosen Datennetzen erfolglose Anmeldeversuche für eine bestimmte, vom Zeitgeber gemessene Zeit aussetzt. Wiederum schont diese Maßnahme sowohl die Ressourcen der Netzbetreiber als auch die des Gerätes.

Die zweite Kontrolleinheit kann vorteilhaft ausgebildet sein, Betriebsparameter wie z. B. die Pause zwischen zwei Anmeldeversuchen, die maximale Anzahl von Anmeldeversuchen und die maximale Zeit, die für einen Anmeldeversuch aufgewendet wird, bevor er als erfolglos abgebrochen wird, vorzugeben und zu verändern. Die anzupassenden Betriebsparameter können automatisch gemäß einem Algorithmus wie einem Pseudozufallsverfahren oder einem sonstigen Verfahren bestimmt werden. In einer besonders bevorzugten Variante dieser Ausführung ist die zweite Kontrolleinheit mit der Mobilfunkschnittstelle mittel- oder unmittelbar verbunden und derartig ausgebildet, dass die Wahl der Parameter vom HMSC aus über die Mobilfunkschnittstelle vorgenommen und ausgelöst werden kann. Dadurch können im Betrieb noch Optimierungen der Betriebsparameter vorgenommen werden, die z. B. ein längeres Batterieleben ermöglichen oder das Gerät an besondere Gegebenheiten eines Aufenthaltslandes anpassen.

In einer bevorzugten Variante des Verfahrens wird zuvor eine erfolglose Anmeldung wegen Zurückweisung durch sämtliche vor Ort verfügbaren Mobilfunk- oder drahtlosen Datennetze erkannt.

In einer besonders bevorzugten Variante der angeführten Verfahren wird eine Liste der lokal empfangbaren Mobilfunk- oder drahtlosen Datennetze erstellt und eine Reihenfolge der einzelnen Einträge der Liste festgelegt. In einer besonders bevorzugten Variante dieses Verfahrens wird die Reihenfolge der Liste der lokal empfangbaren Mobilfunk- oder drahtlosen Datennetze nach der jeweiligen Empfangsstärke bestimmt, so dass Netze höherer Empfangsstärke vorgezogen werden. Durch diese Maßnahme steigt die Wahrscheinlichkeit einer fehlerfreien Datenübertragung, wenn zuerst ein Anmeldeversuch bei einem besser empfangbaren Netz durchgeführt wird und die Anmeldung gelingt.

In einer weiteren Variante des Verfahrens wird nach einer gescheiterten Anmeldung bei einem Netz das gemäß der Reihenfolge nächste Netz der Liste ausgewählt und ein Anmeldeversuch bei diesem Netz veranlasst, selbst wenn dieses Netz eine Anmeldung bei einem vorhergehenden Versuch bereits zurückgewiesen hatte.

Vorzugsweise wird nach erfolglosen Anmeldungen bei allen Netzen der Liste eine neue Liste erstellt. Dadurch werden z. B. durch Ortswechsel während der Ausführung des Verfahrens bedingte Änderungen der Verfügbarkeit von Netzen berücksichtigt.

In einer weiteren bevorzugten Variante des Verfahrens wird nach einer vorgegebenen Zeit ein Anmeldeversuch bei einem Netz als gescheitert angesehen und abgebrochen, wenn keine positive Rückmeldung vom Netz gegeben wurde.

In einer besonders bevorzugten Variante des Verfahrens wird nach einer gescheiterten Anmeldung eine vorgegebene Zeit verstreichen gelassen, bevor ein neuerlicher Anmeldeversuch bei demselben Netz eingeleitet wird. Der Vorteil dieser Maßnahme liegt in der beidseitigen Ressourcenschonung.

In einer weiteren Variante des Verfahrens werden nach einer vorgegebenen Anzahl gescheiterter Anmeldungen eine vorgegebene Zeit lang neuerliche Anmeldeversuche bei demselben oder anderen Netzen ausgesetzt. Auch hier werden wieder sowohl seitens der Netzbetreiber und des Gerätes die jeweiligen Ressourcen geschont.

In allen Verfahrensvarianten wird festgestellt, ob ein Anmeldeversuch bei einem der verfügbaren Netze erfolgreich war, und im Erfolgsfall anschließend das Verfahren beendet.

Die Erfindung soll nun anhand von Ausführungsbeispielen mit Bezug auf die Abbildungen näher erläutert werden. Von den Abbildungen zeigt:
- Abb. 1: ein Blockdiagramm eines Gerätes der mit Anspruch 1 beanspruchten Art;
- Abb. 2: ein Blockdiagramm eines Gerätes der mit Anspruch 1 beanspruchten Art, das mit einer zweiten Telemetriedatenschnittstelle ausgestattet ist;
- Abb. 3: zeigt ein Blockdiagramm einer Variante eines Gerätes der mit Anspruch 1 beanspruchten Art;
- Abb. 4: zeigt ein erweitertes Blockdiagramm als Ausführungsbeispiel einer bevorzugten Ausführungsvariante gemäß der Ansprüche 11, 13 und 14;
- Abb. 5.: zeigt ein Flussdiagramm einer Ausführungsvariante des erfindungsgemäßen Verfahrens;
- Abb. 6: zeigt ein erweitertes Flussdiagramm einer Ausführungsvariante des erfindungsgemäßen Verfahrens;
- Abb. 7: zeigt ein Flussdiagramm einer Ausführungsvariante des beanspruchten Verfahrens mit AT-Befehlen für die einzelnen Schritte

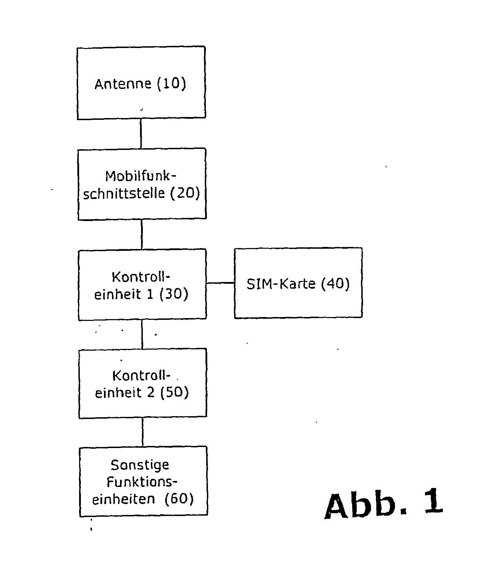

Abb. 1 zeigt beispielhaft ein Blockdiagramm eines Gerätes, wie es mit Anspruch 1 in allgemeiner Form beansprucht wird. In dem Blockdiagramm sind wesentliche Bestandteile des Gerätes aufgeführt. Eine Antenne 10 ist mit einer Mobilfunkschnittstelle 20 verbunden, die von einer ersten Kontrolleinheit 30 gesteuert wird. Die Kontrolleinheit ist mit einer SIM-Karte 40 verbunden, die neben anderen Informationen zum Betrieb der Mobilfunkschnittstelle auch solche zur Identifikation des Benutzers und zum Nachweis dessen Berechtigung zur Benutzung des Mobilfunkdienstes enthält. Erfindungsgemäß ist eine zweite Kontrolleinheit 50 vorgesehen, die mit der Kontrolleinheit 30 verbunden ist. In der in Abb. 1 gezeigten Variante sind sonstige Funktionseinheiten 60, die weitere, nicht näher spezifizierte Funktionen des Gerätes ausführen, mit der zweiten Kontrolleinheit 50 direkt und mit der ersten Kontrolleinheit 30 indirekt verbunden. Es sind auch andere Konfigurationen vorstellbar, bei denen die sonstigen Funktionseinheiten 60 direkt mit der ersten Kontrolleinheit 30 und/oder indirekt mit der zweiten Kontrolleinheit 50 verbunden sind.

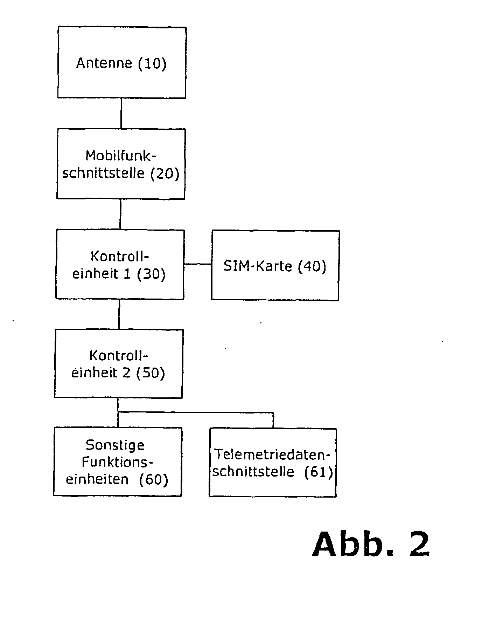

Die Abb. 2 zeigt einen Spezialfall des in Abb. 1 dargestellten Gerätes. Dieses umfasst alle in Abb. 1 enthaltenen Blöcke, die hier nicht noch einmal einzeln aufgeführt werden. Zusätzlich ist eine Telemetriedatenschnittstelle 61 vorgesehen, die bei dem Gerät gemäß Abb. 1 im Block der nicht näher'spezifizierten sonstigen Funktionseinheiten enthalten sein könnte. Diese Telemetriedatenschnittstelle, die nicht notwendigerweise einem der in den Ansprüchen 3 bis 6 genannten drahtlosen Kommunikationsstandards entsprechen muss, kann beispielsweise zur Kommunikation mit einem elektromedizinischen Implantat dienen, so dass das abgebildete Gerät zum Mittler zwischen elektromedizinischem Implantat und Home Monitoring Service Center wird.

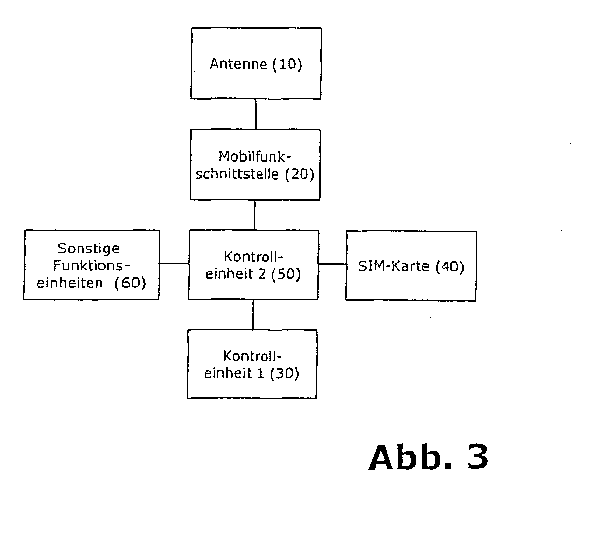

Abb. 3 zeigt eine alternative Ausführung des beanspruchten Gerätes. Die Antenne 10 ist wie zuvor beschrieben mit der Mobilfunkschnittstelle 20 verbunden. Abweichend zu den Geräten gemäß der vorangehenden Darstellungen ist die Mobilfunkschnittstelle 20 hier mit der zweiten Kontrolleinheit 50 verbunden, die wiederum mit der ersten Kontrolleinheit 30, der SIM-Karte 40 und den sonstigen Funktionseinheiten 60 verbunden ist. Die sonstigen Funktionseinheiten 60 können wiederum eine zweite Telemetriedatenschnittstelle enthalten. In dieser alternativen Ausführung liegt die zweite Kontrolleinheit 50 zentral zwischen der Mobilfunkschnittstelle 20, der SIM-Karte 40, der ersten Kontrolleinheit 30 und den sonstigen Funktionseinheiten 60. Sie ist im Sinne der Erfindung derart ausgestaltet, dass sie die Kommunikation zwischen der ersten Kontrolleinheit 30 und der SIM-Karte 40 bzw. zwischen der Mobilfunkschnittstelle 20 und der ersten Kontrolleinheit 30 kontrollieren und filtern kann. Die zweite Kontrolleinheit 50 kann auf diese Weise eine von demjenigen Netz, bei dem eine Anmeldung versucht wird, übermittelte Information über eine Zurückweisung löschen, bevor die erste Kontrolleinheit 30 diese Information auf der SIM-Karte 40 vermerken kann. Alternativ könnte die zweite Kontrolleinheit 50 auch die Übermittelung der ausgewerteten Information über die Zurückweisung an die SIM-Karte 40 von der ersten Kontrolleinheit 30 unterbinden, indem sie die Information nicht an die SIM-Karte 40 weiterleitet. Es sind weitere Varianten vorstellbar, wie die zweite Kontrolleinheit 50 durch Kontrolle, Unterbrechung und Veränderung des Informationsstromes zwischen der SIM-Karte 40, der ersten Kontrolleinheit 30 und der Mobilfunkschnittstelle 20 die Wirkung einer Zurückweisung durch ein Mobilfunknetz aufhebt oder in ihrer Dauer einschränkt. Alle diese Varianten sollen durch den beanspruchten Patentschutz erfasst sein.

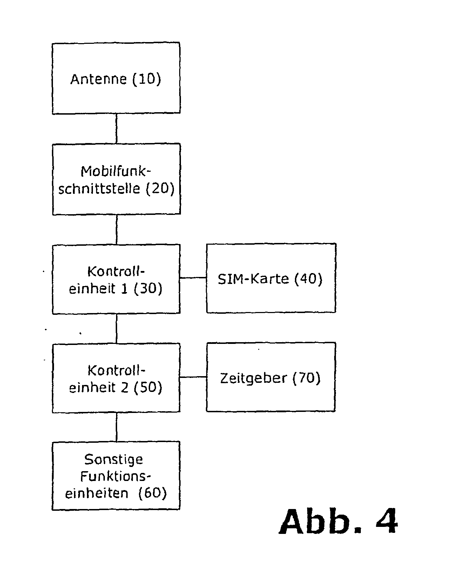

Abb. 4 zeigt ein weiteres Blockdiagramm einer beanspruchten Vorrichtung. Gegenüber dem Gerät gemäß Abb. 1 besitzt die Vorrichtung gemäß Abb. 4 zusätzlich einen Zeitgeber 70, der mit der zweiten Kontrolleinheit 50 verbunden ist. Der Zeitgeber 70 misst die konfigurierbaren Zeiten, die zwischen jedem Schritt des beanspruchten Verfahrens verstreichen gelassen werden sollen und signalisiert der zweiten Kontrolleinheit 50 das Verstreichen der vorgegebenen Zeiten auf geeignete Weise.

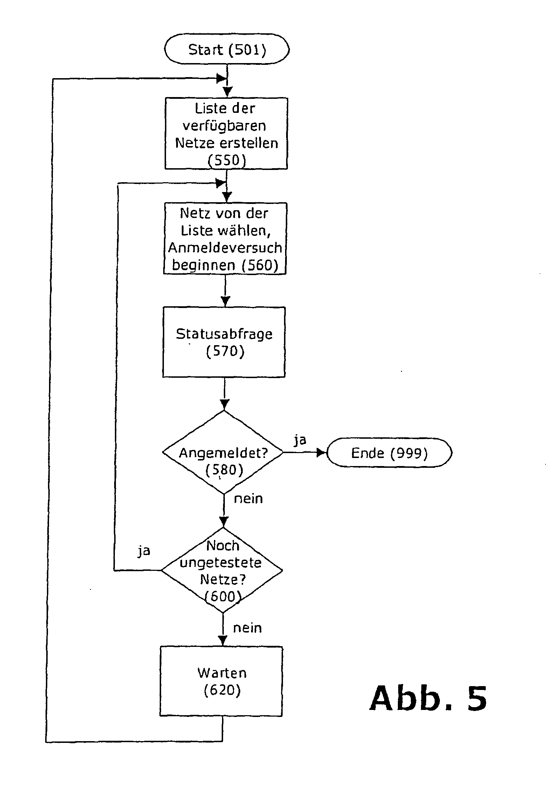

Die Abbildungen 5 bis 7 zeigen Flussdiagramme als Beispiele der beanspruchten Verfahren. Bei allen Beschreibungen von Flussdiagrammen geben die Zahlen in Klammern den entsprechenden Verfahrensschritt gemäß der Nummerierung in den Diagrammen an, auf den sich die Ausführungen beziehen. Dies geschieht immer dort, wo der Schritt nicht explizit im Satz angeführt wird.

Abb. 5 zeigt ein Flussdiagramm eines Ausführungsbeispiels des beanspruchten Verfahrens. Nach dem Start (501) erfolgt als erster Schritt ein Erstellen einer Liste von am jeweiligen Ort verfügbaren Netzen (550). Im nächsten Schritt wird ein Anmeldeversuch bei einem ausgewählten Netz aus der Liste begonnen (560). Anschließend wird der Status der Mobilfunkschnittstelle abgefragt (570), um zu kontrollieren, ob der Anmeldeversuch erfolgreich war (580). War der Anmeldeversuch erfolgreich, wird das Verfahren beendet (999). Andernfalls wird geprüft, ob die Liste der vor Ort verfügbaren Netze noch weitere Netze enthält, bei denen seit Erstellen der aktuell verwendeten Liste noch kein Anmeldeversuch unternommen wurde (600). Weist die Liste solche Netze auf, so wird zum Schritt 560 zurückverzweigt, eines dieser Netze ausgewählt und wie gehabt weiterverfahren. Enthält die Liste keine weiteren Netze, wird nach einer optionalen Wartepause 620 zum Schritt 550 zurückverzweigt und eine neue Liste erstellt. Das Verfahren setzt sich entsprechend fort, bis es mit dem Erfolgsfall beendet wird (999).

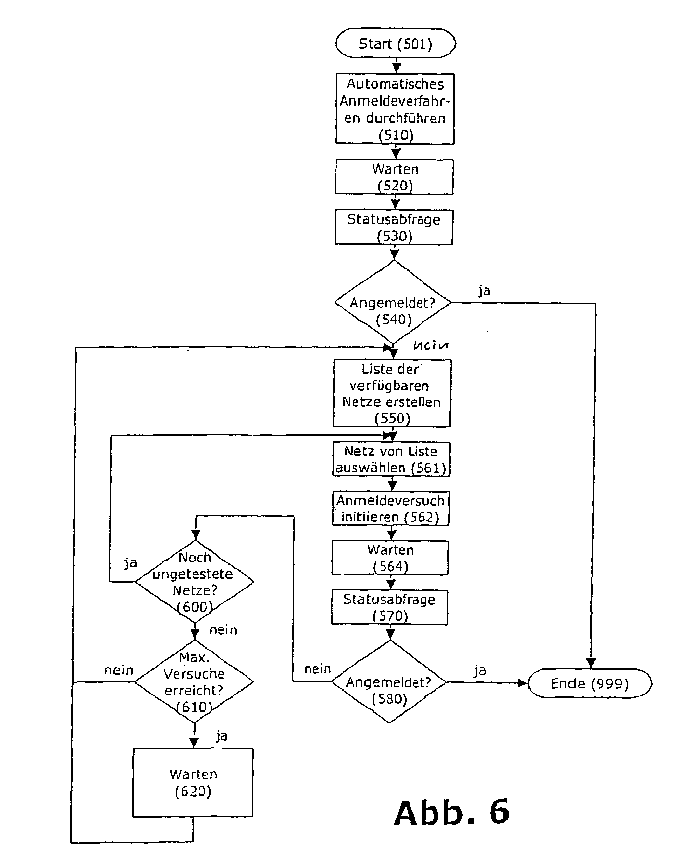

Abb. 6 zeigt beispielhaft eine erweiterte Variante des beanspruchten Verfahrens. Beginnend beim Start 501 wird zuerst das normale automatisierte Anmeldeverfahren durchgeführt (510). Daraufhin wird eine bestimmte Zeit abgewartet (520) und anschließend der Status abgefragt (530). War das automatische Anmeldeverfahren erfolgreich (540), wird das Verfahren erfolgreich beendet (999). Andernfalls wird eine Liste der vor Ort verfügbaren Netze erstellt (550) und ein Netz von der Liste ausgewählt (561). Bei diesem Netz wird in Schritt 562 ein Anmeldeversuch initiiert und anschließend eine bestimmte, vorgebbare Zeit abgewartet (564). Anschließend wird eine Statusabfrage durchgeführt (570) und das Ergebnis überprüft (580). Ist das Gerät bei dem gewählten Netz erfolgreich angemeldet worden, dann wird das Verfahren beendet (999). Ist das Gegenteil der Fall, wird die verwendete Liste auf weitere vorhandene ungetestete Netze geprüft (600). Sind solche Netze noch vorhanden, wird zum Schritt 561 zurückverzweigt und entsprechend weiterverfahren. Enthält die Liste keine weiteren ungetesteten Netze, wird geprüft, ob eine vorgegebene maximale Zahl von Anmeldeversuchen erreicht wurde (610). Diese maximale Zahl von Anmeldeversuchen kann sich auf die Anzahl der insgesamt durchgeführten Anmeldeversuche beziehen oder aber auf die Anzahl der pro Netz durchgeführten Anmeldeversuche bzw. die Anzahl der abgearbeiteten Listen. Ist die maximale Anzahl erreicht, werden die Anmeldeversuche eine vorgegebene Zeit ausgesetzt (620, anschließend zurück zu 550), andernfalls wird direkt oder nach einer kürzeren Pause als bei Erreichen der maximalen Anzahl zu Schritt 550 zurückverzweigt. Eine Variante dieses Verfahrens sieht vor, dass auch eine Prüfung der maximalen Anzahl von Anmeldeversuchen für jedes Netz einzeln durchgeführt wird und dieses Netz bei Erreichen der maximalen Anzahl von Anmeldeversuchen eine vorgegebene Zeit lang von weiteren Anmeldeversuchen ausgenommen wird. Dies ist dann sinnvoll, wenn die Listen z. B. aufgrund von Ortswechseln variieren, so dass sie eine wechselnde Auswahl an Netzen aufführen. Nach dem Erstellen der Liste der verfügbaren Netze (550) werden in einem zusätzlichen Schritt solche Netze aus der Liste gestrichen, bei denen schon die vorgegebene maximale Anzahl von Anmeldeversuchen erreicht wurde. Nach Ablauf einer für jedes Netz einzeln gemessenen Zeit werden die Netze dann wieder, so sie vor Ort verfügbar sind, in die erstellten Listen aufgenommen.

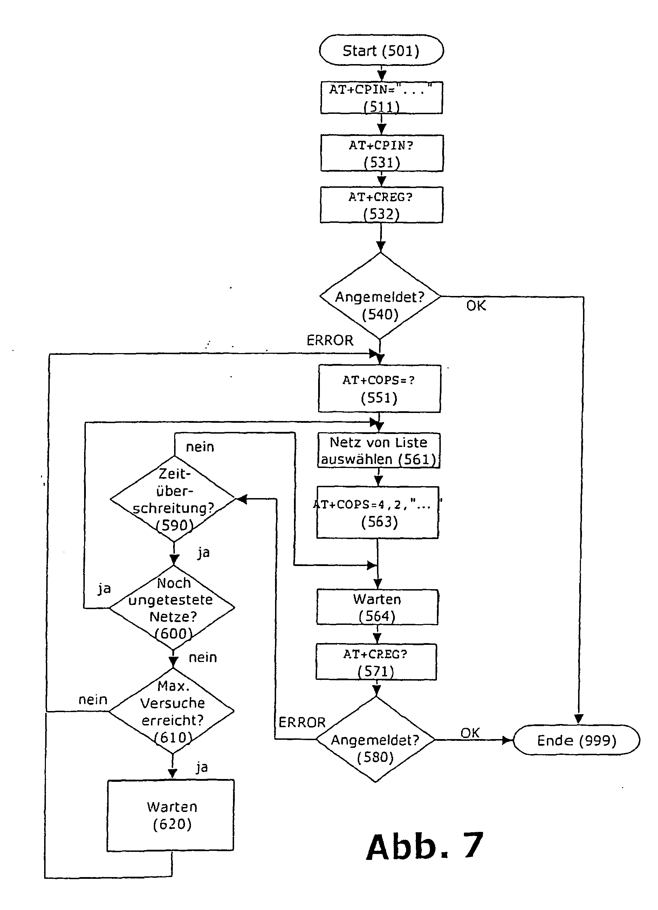

Abb. 7 zeigt ein Flussdiagramm eines ähnlichen Verfahrens wie es in Abb. 6 dargestellt ist. Hier wurden jedoch AT-Befehle als Beispiele angegeben, um die Steuerung der ersten Kontrolleinheit (30 in den Abb. 1 bis 4) einer Vorrichtung nach Anspruch 13 zu erläutern. Dort, wo abhängig vom Ergebnis eines AT-Befehls verzweigt wird, sind typische Antworten "OK" im Erfolgsfall und "ERROR" im Fehlerfall. Startend bei 501 wird in Schritt 511 der Befehl AT+CPIN="PIN-Code" ausgegeben. "PIN-Code" bedeutet den sogenannten PIN-Code der SIM-Karte, der im AT-Befehl in doppelte Anführungszeichen eingefasst wird (z. B.: "1234"). Dieser Befehl veranlasst die empfangende Kontrolleinheit dazu, den PIN-Code an die SIM-Karte zu übermitteln, welche ihn mit der intern gespeicherten Kopie vergleicht. Dieser Prozess ist nicht mit der Übermittlung der geheimen Benutzeridentifikationsinformation, die auf der SIM-Karte abgelegt ist, an das HLR zu verwechseln! Bei der Prüfung des PIN-Codes handelt es sich lediglich um einen Sicherungsmechanismus, der eingeführt wurde, um das Gerät vor Benutzung durch andere als den Eigentümer zu schützen. Der PIN-Code kann deshalb auch gewöhnlich von diesem gewählt werden. Das Ergebnis der Prüfung des PIN-Codes, der hier nicht notwendigerweise vom Benutzer gewählt und eingegeben wird, sondern vielmehr direkt in der aus AT-Befehlen bestehenden Steuersequenz der Kontrolleinheiten enthalten ist, wird im Schritt 531 mit dem AT-Befehl AT+CPIN? abgefragt. Die Eingabe des PIN-Codes löst bei Mobilfunkschnittstellen, die nach dem Stand der Technik ausgeführt sind, normalerweise ein automatisches Anmeldeverfahren aus. Dessen vollständige Durchführung kann durch eine in der Abbildung nicht gezeigte Pause zwischen den Schritten 531 und 532 abgewartet werden. Es wird mit Schritt 532 weiterverfahren, indem mit dem AT-Befehl AT+CREG? der Anmeldestatus abfragt wird. Wurde das Gerät schon im automatischen Anmeldeverfahren erfolgreich angemeldet, folgt der Prüfung in 540 direkt das Ende des Verfahrens (999). Andernfalls wird die empfangende Kontrolleinheit in Schritt 551 durch den AT-Befehl AT+COPS=? angewiesen, eine Liste der empfangbaren Netze zu erstellen und auszugeben. In Schritt 561 wird ein Netz von der Liste ausgewählt und eine Anmeldung bei diesem Netz in 563 durch einen AT-Befehl wie z. B. AT+COPS=4,2, "26201" initiiert. Der dritte Befehlsparameter bestimmt dabei durch die fünfstellige Netzkennung das jeweilige Netz, bei dem die Anmeldung versucht werden soll. Im Schritt 564 wird ggf. eine vorgegebene Zeit abgewartet, bevor im Schritt 571 mit AT+CREG? der Anmeldestatus abgefragt wird. Der Schritt 564 kann entfallen, wenn die im Schritt 571 ausgelöste Statusabfrage selbst bereits eine Wartezeit beinhaltet. Ist das Gerät erfolgreich angemeldet worden (580), wird das Verfahren beendet (999). Andernfalls wird überprüft, ob es bei der Anmeldung bereits zu einer Überschreitung einer vorgegebenen Maximalzeit für die Anmeldung gekommen ist (590). Ist keine Zeitüberschreitung eingetreten, wird zu Schritt 564 zurückverzweigt und wiederum gewartet. Kam es jedoch zu einer Zeitüberschreitung, dann war der Anmeldeversuch erfolglos und es wird in Schritt 600 geprüft, ob die verwendete Liste der verfügbaren Netze noch aktuell ungeprüfte Einträge enthält. Gibt es noch weitere solcher Netze, wird zu Schritt 561 zurückgesprungen und ein Anmeldeversuch bei einem der ungeprüften Netze unternommen. Enthält die Liste keine weiteren ungetesteten Netze mehr, wird geprüft, ob eine vorgegebene maximale Anzahl von Anmeldeversuchen erreicht wurde. Diese maximale Anzahl von Anmeldeversuchen kann sich, wie schon in den Ausführungen zur Abb. 6 erklärt, sowohl auf die Anzahl der Anmeldeversuche bei jedem einzelnen Netz innerhalb eines bestimmten Zeitraumes als auch auf die Anzahl der seit der letzten Aussetzung oder dem Beginn des Anmeldeverfahrens abgearbeiteten Listen der vor Ort verfügbaren Netze beziehen. Wurde die maximale Anzahl von Anmeldeversuchen erreicht, werden die Anmeldeversuche durch Warten ausgesetzt (620), andernfalls wird nach gar keiner oder einer kürzeren Pause als in 620 zum Schritt 551 zurückgesprungen und mit einer neuen Liste weiterverfahren.

## Patentansprüche

1. Vorrichtung mit
wenigstens einer Telemetriedatenschnittstelle, die als Mobilfunkschnittstelle ausgelegt ist, einen Datenaustausch über ein Mobilfunk- oder drahtloses Datennetz durchzuführen,
einem Benutzeridentifikationsmodul, das Informationen zum Betrieb enthält, die unter anderem die Berechtigung eines Benutzers zur Benutzung von Mobilfunk- oder drahtlosen Datennetzen ausweisen,
einer ersten Kontrolleinheit, die auf Basis der auf dem Benutzeridentifikationsmodul gespeicherten Informationen Zustandekommen und Durchführung des Datenaustausches steuert und die nach Empfang einer seitens eines örtlich verfügbaren Mobilfunk- oder drahtlosen Datennetzes empfangenen Information über eine Zurückweisung von sich aus alle weiteren Versuche, eine Verbindung über ein drahtloses Kommunikationsnetz herzustellen, einstellt, und
einer zweiten Kontrolleinheit, die ausgebildet ist, die Zurückweisung des Benutzeridentifikationsmoduls infolge der empfangenen Information über die Zurückweisung bei der Anmeldung zum Mobilfunk- oder drahtlosen Datennetz durch geeignete Steuerung der ersten Kontrolleinheit im weiteren Verlauf des Anmeldeverfahrens durch Umgehen oder Löschen der Information über die Zurückweisung unwirksam zu machen oder in der Dauer ihrer Wirkung zu beschränken, so dass die Vorrichtung nach vorangegangener Zurückweisung durch die örtlich verfügbaren Mobilfunk- oder drahtlosen Datennetze einen Datenaustausch über diese Mobilfunk- oder drahtlosen Datennetze durchführen kann, wenn die Ursache der Zurückweisung nicht mehr besteht,
die erste Kontrolleinheit, die ausgebildet ist, eine Liste der lokal empfangbaren Mobilfunk- oder drahtlosen Datennetze zu erstellen und der zweiten Kontrolleinheit zugänglich zu machen, und
die zweite Kontrolleinheit, die ausgebildet ist,
- nach einer gescheiterten Anmeldung bei einem Netz ein weiteres Netz von der Liste auszuwählen und die erste Kontrolleinheit zu einem Anmeldeversuch bei diesem Netz zu veranlassen, selbst wenn dieses Netz eine Anmeldung bei einem vorhergehenden Versuch bereits zurückgewiesen hatte, und
**dadurch gekennzeichnet ist**,
- nach erfolglosen Anmeldeversuchen bei allen Netzen der Liste eine neue Liste von der ersten Kontrolleinheit erstellen zu lassen.

2. Vorrichtung nach Anspruch 1, **dadurch gekennzeichnet,**
**dass** sie durch eine Person tragbar ist.

3. Vorrichtung nach einem der vorrangehenden Ansprüche, **dadurch gekennzeichnet,**
**dass** sie über wenigstens eine zweite Telemetriedatenschnittstelle verfügt, über die sie mit einem elektromedizinischen Implantat,
insbesondere einem Herzschrittmacher, kommunizieren kann,
und durch Auswerten und/oder Weiterleiten der von dem elektromedizinischen Implantat empfangenen technischen und medizinischen Daten über die erste Telemetriedatenschnittstelle zu einem Home Monitoring Service Center ein Home Monitoring eines Patienten bzw. der Funktion eines ihm implantierten elektromedizinischen Implantats ermöglicht.

4. Vorrichtung nach einem der vorhergehenden Ansprüche, **dadurch gekennzeichnet,**
**dass** die Mobilfunkschnittstelle ausgebildet ist, den Datenaustausch über ein GSM-, UMTS-, CDMA- oder WLAN-Netz durchzuführen.

5. Vorrichtung nach einem der vorhergehenden Ansprüche, **dadurch gekennzeichnet,**
**dass** mindestens eine der Kontrolleinheiten ausgebildet ist, eine erfolglose Anmeldung wegen Zurückweisung durch sämtliche vor Ort verfügbaren Mobilfunk- oder drahtlosen Datennetze zu erkennen.

6. Vorrichtung nach einem der vorhergehenden Ansprüche, **dadurch gekennzeichnet,**
**dass** die zweite Kontrolleinheit einen Zeitgeber enthält oder mit einem solchen verbunden ist und derart ausgebildet ist, dass sie nach einer gescheiterten Anmeldung eine vorgegebene, durch den Zeitgeber gemessene Zeitdauer verstreichen lässt, bevor sie einen neuerlichen Anmeldeversuch bei demselben Netz einleitet.

7. Vorrichtung nach einem der vorhergehenden Ansprüche, **dadurch gekennzeichnet,**
**dass** die Kontrolleinheiten ausgebildet sind, AT-Befehle auszugeben und/oder auszuführen.

8. Vorrichtung nach einem der vorhergehenden Ansprüche, **dadurch gekennzeichnet,**
**dass** die zweite Kontrolleinheit einen Zeitgeber enthält oder mit einem solchen verbunden ist und derartig ausgebildet ist, dass sie wiederholte, bei allen verfügbaren Mobilfunk- oder drahtlosen Datennetzen erfolglose Anmeldeversuche für eine bestimmte, vom Zeitgeber gemessene Zeit aussetzt.

9. Vorrichtung nach einem der vorhergehenden Ansprüche, **dadurch gekennzeichnet,**
**dass** die zweite Kontrolleinheit einen Zeitgeber und einen Zähler enthält oder mit solchen verbunden ist und ausgebildet ist, Betriebsparameter wie z. B. die Pause zwischen zwei Anmeldeversuchen, die maximale Anzahl von Anmeldeversuchen und die maximale Zeit, die für einen Anmeldeversuch aufgewendet wird, bevor er als erfolglos abgebrochen wird, vorzugeben und zu verändern.

10. Vorrichtung nach Anspruch 9, **dadurch gekennzeichnet,**
**dass** die zweite Kontrolleinheit mit der Mobilfunkschnittstelle verbunden und derartig ausgebildet ist, dass die Wahl der Parameter vom Home Monitoring Service Center aus über die Mobilfunkschnittstelle vorgenommen und ausgelöst werden kann.

## Claims

1. A device having
at least one telemetry data interface, which is designed as a mobile radio interface, to perform a data exchange via a mobile radio or wireless data network,
a user identification module, which contains information on the operation, which proves, *inter alia*, the authorization of a user to use mobile radio or wireless data networks,
a first control unit, which controls the establishment and performance of the data exchange on the basis of the information stored on the user identification module and which, after receiving information about a rejection on the part of a locally available mobile radio or wireless data network, stops all further attempts to produce a connection via a wireless communication network, and
a second control unit, which is implemented to make the rejection of the user identification module as a result of the received information about the rejection upon the sign-in to the mobile radio or wireless data network inactive or to restrict it in the duration of its action by suitable control of the first control unit in the further course of the sign-in method by bypassing or erasing the information about the rejection, so that the device, after prior rejection by the locally available mobile radio or wireless data networks, may perform a data exchange via these mobile radio or wireless data networks, if the cause of the rejection no longer exists,
the first control unit, which is implemented to prepare a list of the locally receivable mobile radio or wireless data networks and make it accessible to the second control unit, and
the second control unit, which is implemented,
- after a failed sign-in with a network, to select a further network from the list and to prompt the first sign-in unit to a sign-in attempt with this network, even if this network has already rejected a sign-in during a preceding attempt, and is **characterized in that**,
- after unsuccessful sign-in attempts with all networks of the list, it has a new list prepared by the first control unit.

2. The device according to Claim 1, **characterized in that** it is portable by a person.

3. The device according to one of the preceding claims, **characterized in that** it has at least one second telemetry data interface, via which it may communicate with an electromedical implant,
in particular a cardiac pacemaker,
and, by analyzing and/or relaying the technical and medical data received from the electromedical implant via the first telemetry data interface to a home monitoring service center, allows home monitoring of a patient and/or the function of an electromedical implant implanted therein.

4. The device according to one of the preceding claims, **characterized in that** the mobile radio interface is implemented to perform the data exchange via a GSM, UMTS, CDMA, or WLAN network.

5. The device according to one of the preceding claims, **characterized in that** at least one of the control units is implemented to recognize an unsuccessful sign-in because of rejection by all of the locally available mobile radio or wireless data networks.

6. The device according to one of the preceding claims, **characterized in that** the second control unit contains a timer or is connected thereto and is implemented in such a way that it permits a predefined period of time measured by the timer to pass after a failed sign-in before it initiates a new sign-in attempt with the same network.

7. The device according to one of the preceding claims, **characterized in that** the control units are implemented to output and/or execute AT commands.

8. The device according to one of the preceding claims, **characterized in that** the second control unit contains a timer or is connected thereto and is implemented in such a way that it suspends repeated unsuccessful sign-in attempts with all of available mobile radio or wireless data networks for a specific time measured by the timer.

9. The device according to one of the preceding claims, **characterized in that** the second control unit contains a timer and a counter or is connected thereto and is implemented to predefine and change operating parameters such as the pause between two sign-in attempts, the maximum number of sign-in attempts, and the maximum time which is applied for a sign-in attempt before it is terminated as unsuccessful.

10. The device according to Claim 9, **characterized in that** the second control unit is connected to the mobile radio interface and is implemented in such a way that the selection of the parameters may be performed and triggered from the home monitoring service center via the mobile radio interface.

## Revendications

1. Dispositif comprenant
au moins une interface de données de télémétrie, qui est conçue comme interface de téléphonie mobile pour effectuer un échange de données par l'intermédiaire d'un réseau de téléphonie mobile ou d'un réseau de données sans fil,
un module d'identification d'utilisateur, qui contient des informations sur le fonctionnement qui mentionnent entre autres l'autorisation d'un utilisateur pour l'utilisation de réseaux de téléphonie mobile ou de réseau de données sans fil,
une première unité de contrôle, qui commande sur la base des informations mémorisées sur le module d'identification d'utilisateur la survenance et l'exécution de l'échange de données et qui, après la réception d'une information reçue d'un réseau de téléphonie mobile ou d'un réseau de données sans fil localement disponible et relative à un refus, effectue automatiquement tous les autres essais pour établir une liaison par un réseau de communication sans fil, et
une seconde unité de contrôle, qui est conçue pour rendre inefficace le refus du module d'identification d'utilisateur à la suite de l'information reçue relative au refus lors de la demande auprès du réseau de téléphonie mobile ou du réseau de données sans fil par une commande appropriée de la première unité de contrôle au cours du déroulement de la procédure de demande par contournement ou suppression de l'information relative au refus ou pour limiter son effet dans la durée, de telle sorte que le dispositif peut effectuer après un refus préalable par les réseaux de téléphonie mobile ou les réseaux de données sans fil localement disponibles un échange de données par ces réseaux de téléphonie mobile ou réseaux de données sans fil lorsque la cause du refus n'existe plus,
la première unité de contrôle qui est conçue pour établir une liste des réseaux de téléphonie mobile ou des réseaux de données sans fil pouvant recevoir localement et les rendre accessibles à la seconde unité de contrôle, et
la seconde unité de contrôle qui est conçue pour
- sélectionner un autre réseau dans la liste après un échec de demande auprès d'un réseau et pour demander à la première unité de contrôle un essai de demande auprès de ce réseau, même si ce réseau avait déjà refusé une demande lors d'une tentative précédente, et pour
- faire établir une nouvelle liste par la première unité de contrôle après des essais de demande infructueux auprès de tous les réseaux de la liste.

2. Dispositif selon la revendication 1, **caractérisé en ce que**
il peut être porté par une personne.

3. Dispositif selon l'une quelconque des revendications précédentes, **caractérisé en ce que**
il dispose d'au moins une seconde interface de données de télémétrie, par laquelle il peut communiquer avec un implant électromédical,
en particulier avec un pacemaker,
et, par l'analyse et/ou la transmission des données techniques et médicales reçues de l'implant électromédical par l'intermédiaire de la première interface de données de télémétrie à un centre de service de surveillance à domicile (Home Monitoring Service Center), permet d'avoir une surveillance à domicile (Home Monitoring) d'un patient et/ou de la fonction d'un implant électromédical implanté dans lui.

4. Dispositif selon l'une quelconque des revendications précédentes, **caractérisé en ce que**
l'interface de téléphonie mobile est conçue pour effectuer l'échange de données par l'intermédiaire d'un réseau GSM, UMTS, CDMA ou WLAN.

5. Dispositif selon l'une quelconque des revendications précédentes, **caractérisé en ce que**
au moins l'une des unités de contrôle est conçue pour reconnaître une demande infructueuse due à un refus par tous les réseaux de téléphonie mobile ou réseaux de données sans fil qui sont disponibles sur place.

6. Dispositif selon l'une quelconque des revendications précédentes, **caractérisé en ce que**
la seconde unité de contrôle contient un temporisateur ou est reliée avec un tel dispositif et est conçue de telle sorte que, à un échec de demande, elle laisse s'écouler une durée prédéfinie et mesurée par le temporisateur avant qu'elle enclenche une nouvelle tentative de demande auprès du même réseau.

7. Dispositif selon l'une quelconque des revendications précédentes, **caractérisé en ce que**
les unités de contrôle sont conçues pour éditer et/ou exécuter des instructions AT.

8. Dispositif selon l'une quelconque des revendications précédentes, **caractérisé en ce que**
la seconde unité de contrôle contient un temporisateur ou est reliée à un tel dispositif et est conçue de telle sorte qu'elle suspend des essais de demande répétés et infructueux effectués auprès de tous les réseaux de téléphonie mobile ou réseaux de données sans fil disponibles pour une durée définie et mesurée par le temporisateur.

9. Dispositif selon l'une quelconque des revendications précédentes, **caractérisé en ce que**
la seconde unité de contrôle contient un temporisateur et un compteur ou est reliée à ces dispositifs et est conçue pour prédéfinir et modifier des paramètres de service comme par exemple la pause entre deux essais de demande, le nombre maximum d'essais de demande et le temps maximal utilisé pour un essai de demande avant qu'il soit interrompu sans résultat.

10. Dispositif selon la revendication 9, **caractérisé en ce que**
la seconde unité de contrôle est reliée à l'interface de téléphonie mobile et est conçue de telle sorte que le choix des paramètres peut être effectué et déclenché par le centre de service de surveillance à domicile (Home Monitoring Service Center) par l'intermédiaire de l'interface de téléphonie mobile.
